# EUROPÄISCHE PATENTSCHRIFT

(11) **EP 2 260 647 B1**
(45) Veröffentlichungstag und Bekanntmachung des Hinweises auf die Patenterteilung: **31.07.2013**
(21) Anmeldenummer: 09761475.4
(22) Anmeldetag: 10.06.2009
(51) Int. Cl.: H04N 9/04, H04N 9/64

(54) **VERFAHREN UND ENDOSKOP ZUR VERBESSERUNG VON ENDOSKOPBILDERN**
METHOD AND ENDOSCOPE FOR IMPROVING ENDOSCOPE IMAGES
PROCÉDÉ ET ENDOSCOPE DESTINÉS À AMÉLIORER LES IMAGES ENDOSCOPIQUES

(30) Priorität: 12.06.2008 DE 102008027905
(43) Veröffentlichungstag der Anmeldung: 15.12.2010
(73) Patentinhaber: OLYMPUS WINTER & IBE GMBH, 22045 Hamburg (DE)
(72) Erfinder: STEHLE, Thomas, 52064 Aachen (DE); AACH, Til, 52072 Aachen (DE)
(74) Vertreter: Hausfeld, Norbert
(86) Internationale Anmeldenummer: PCT/EP2009/004171
(87) Internationale Veröffentlichungsnummer: WO 2009/149912

(56) Entgegenhaltungen:
- DE-C1- 19 902 184
- US-A- 4 768 089
- US-A- 5 092 331

## Beschreibung

Die Erfindung betrifft ein Verfahren nach dem Oberbegriff des Anspruches 1 sowie ein Endoskop nach dem Oberbegriff des Anspruches 4.

Endoskopische Bilder fluoreszierender Gewebebereiche im menschlichen Körper werden zur Entdeckung entsprechend markierter Tumore verwendet, z. B. in der Wand der Blase. Aus der DE 199 02 184 C 1 ist es bekannt, dabei das betrachtete Gewebe mit einem Hintergrundlicht zu beleuchten, das in der Wellenlänge vom Fluoreszenzlicht deutlich entfernt ist, also gut diesem gegenüber unterscheidbar ist und auch in der Helligkeit niedrig liegt, um das sehr schwache Fluoreszenzlicht nicht zu überstrahlen. Dennoch ist die Erkennung der sehr schwachen Fluoreszenz stets schwierig. Verfahren zur Verbesserung solcher Endoskopbilder waren bisher wenig erfolgreich.

Verfahren zur Bildverbesserung bei endoskopischen Bildern mittels Farbraumtransformation sind aus der US 4 805 016 A bekannt.

Aus der US 4,768,089 A ist ein Verfahren zur Verbesserung von aufgenommenen Endoskopbildern bekannt, bei der eine Transformierung aus dem Farbraum RGB der Videokamera in den Farbraum "hue-intensity-saturation" (Farbton-Intensität-Sättigung) und eine Farbtonverarbeitung erfolgt. Es soll die Erkennbarkeit der Fluoreszenz verbessert werden.

Die Aufgabe der vorliegenden Erfindung besteht darin, bei den eingangs erwähnten endoskopisch gewonnenen Fluoreszenzbildern die Erkennbarkeit der Fluoreszenz zu verbessern.

Diese Aufgabe wird mit den Merkmalen der Kennzeichnungsteile der Ansprüche 1 sowie 4 gelöst.

Die mittels einer Videokamera gewonnenen Endoskopbilder liegen zumeist im üblichen RGB-Farbraum vor. Entsprechend der Aufgabe der Erfindung soll bei diesen Bildern die Fluoreszenz beeinflusst werden, wobei aber nach Möglichkeit die sonstigen Bildeigenschaften unverändert bleiben sollen. Dazu werden zunächst die Farbpixel in einen Farbraum transformiert, in dem eine für die Fluoreszenz charakteristische Gerade, die einerseits durch den Farbraumbereich des Hintergrundlichtes und andererseits durch den Farbraumbereich des Fluoreszenzlichtes verläuft, parallel zu einer Koordinatenachse dieses Farbraumes ausgerichtet ist. Dann kann in diesem Farbraum, durch Verstellen entlang dieser, die Fluoreszenz beschreibenden Koordinatenachse die Fluoreszenz beeinflusst werden, ohne sonstige Bildeigenschaften zu verändern. Nun kann mit einer nichtlinearen Kennlinie die Fluoreszenzkomponente der Pixel im Sinne einer Kontrasterhöhung der Fluoreszenzwerte umgerechnet werden. Anschließend werden die Pixel wieder in einen zur Bilddarstellung geeigneten Farbraum, z. B. den üblichen RGB-Farbraum, rücktransformiert.

Vorteilhaft ist gemäß Anspruch 2 die Kennlinie derart ausgebildet, dass sie im mittleren Bereich in einem oberen Abschnitt die Fluoreszenzwerte anhebt und in einem unteren Abschnitt absenkt, wobei in den Endbereichen der Kennlinie die Fluoreszenz unverändert bleibt. Es ergibt sich somit eine Kennlinie, die in den oberen und unteren Endbereichen auf der Identitätsgeraden liegt und dazwischen im Wesentlichen S-förmig ausgebildet ist.

Vorteilhaft sind die Merkmale des Anspruches 3 gekennzeichnet. Auf diese Weise lässt sich der Fluoreszenzkontrast mit fortschreitender Zeit vergrößern. Damit kann das allmähliche Ausbleichen der fluoreszierenden Substanz, das im Laufe der Zeit zu einem immer geringeren Fluoreszenzkontrast führt, ausgeglichen werden.

Ein erfindungsgemäßes medizinisches Endoskop ist in Anspruch 4 angegeben. Dieses Endoskop arbeitet erfindungsgemäß bezüglich der Bildverarbeitungseinrichtung nach einem der in den Ansprüchen 1 bis 3 angegebenen Verfahren.

In den Zeichnungen ist die Erfindung beispielsweise und schematisch dargestellt. Es zeigen:
- Fig. 1: eine schematische Ansicht eines Endoskops mit Bildverarbeitungseinrichtung und Bilddarstellungseinrichtung,
- Fig. 2: eine vergrößerte schematische Darstellung der Bildverarbeitungseinrichtung,
- Fig. 3: ein Diagramm der verwendeten Fluoreszenzkennlinie,
- Fig. 4: ein Diagramm der verwendeten Lichtkomponenten und
- Fig. 5: eine Darstellung der Bildpixel im RGB-Farbraum.

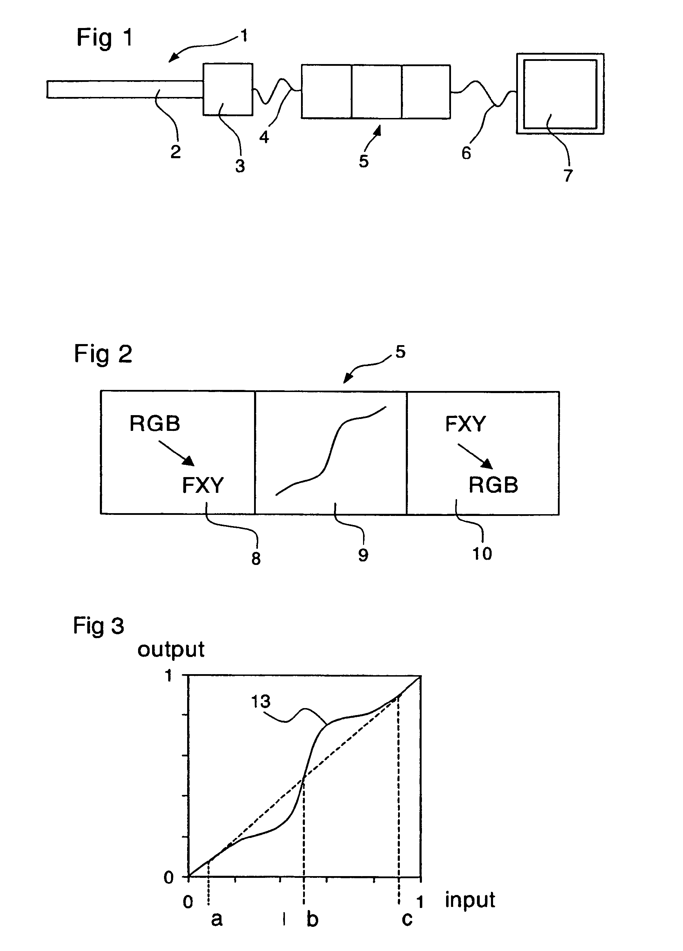

Fig. 1 zeigt ein medizinisches Endoskop 1 mit einem langgestreckten Schaft 2, an dessen proximalem Ende eine Farb-Videokamera 3 angeordnet ist. In anderer Ausführung kann die Kamera 3 auch im distalen Endbereich des Schaftes 2 unmittelbar hinter dem dort vorgesehenen Objektiv angeordnet sein.

Die Farb-Videokamera 3 ist mit einer Leitung 4, die zur Datenübertragung und beispielsweise auch zur Stromversorgung dient, mit einer Bildverarbeitungseinrichtung 5 verbunden, um dieser Bilddaten zuzuführen. Die Bildverarbeitungseinrichtung 5 ist mit einer Leitung 6 an eine Bilddarstellungseinrichtung 7 angeschlossen, z. B. einen handelsüblichen Monitor.

Das Endoskop 1 kann beispielsweise in der Urologie zur Untersuchung der Blasenwand auf Tumore verwendet werden und wird dazu mit seinem Schaft 2 durch die Uretra bis in die Blase (nicht dargestellt) eingeführt. Dabei wird von der Videokamera 3 das betrachtete Bild aufgenommen, an die Bildverarbeitungseinrichtung 5 übertragen, dort aufbereitet und sodann auf der Bilddarstellungseinrichtung 7 zur Anzeige gebracht.

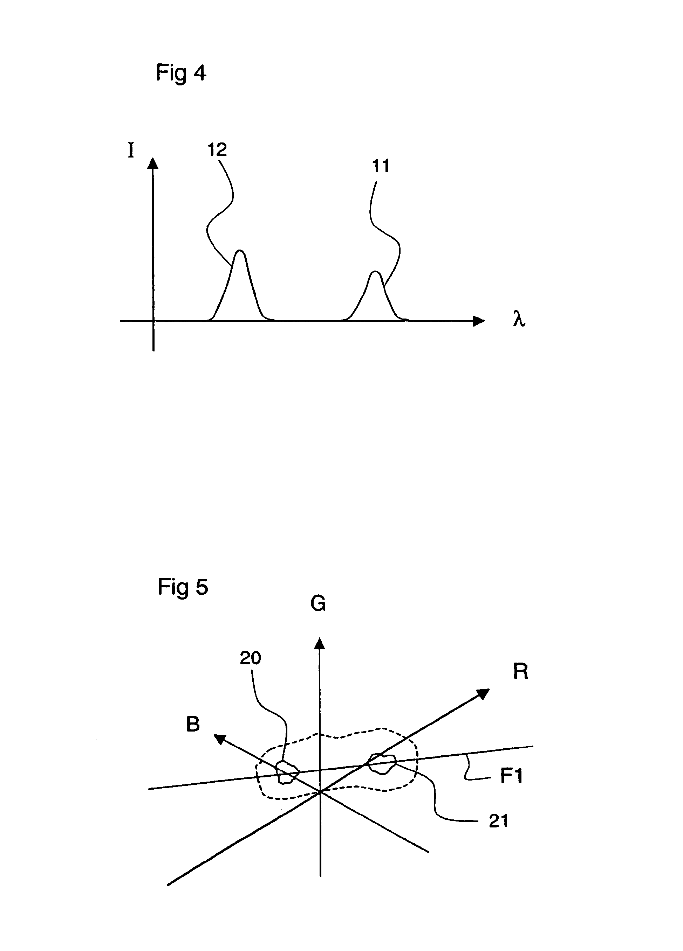

Das Endoskop 1 dient zur Untersuchung von Gewebeoberflächen, z. B. der Blasenwand, auf vorhandene Tumore, die mit einer fluoreszierenden Substanz markiert sind. Wie in Fig. 4 dargestellt, in der die Lichtintensität I über der Wellenlänge λ aufgetragen ist, strahlt fluoreszierendes Gewebe Licht im Bereich 11 ab. Die gesamte betrachtete Oberfläche ist mit Hintergrundlicht im Bereich 12, also bei einer anderen Wellenlänge, beleuchtet. Der Bereich 11 liegt üblicherweise im Roten und der Bereich 12 im Blauen. Zu Einzelheiten hierzu wird auf die DE 199 02 184 C 1 verwiesen.

Die Pixel eines von der Videokamera 3 aufgenommenen Bildes liegen im RGB-Farbraum in einer Wolke, wie sie beispielsweise in Fig. 5 mit gestricheltem Umriss dargestellt ist. Diese Wolke hat charakteristische Schwerpunkte in Farbraumbereichen 20 und 21, die in Figur 5 in einem Beispiel dargestellt sind. Der Farbraumbereich 21 liegt im Roten und entspricht dem Fluoreszenzlicht im Bereich 11 der Fig. 4. Der Farbraumbereich 20 liegt im Blauen im Bereich 12 der Figur 4.

In Fig. 5 ist mit F1 eine Gerade dargestellt, die durch die Farbraumbereiche 20 und 21 verläuft und normalerweise schief zu allen Koordinatenachsen R, G, B verläuft. Die Gerade F1 verläuft durch den Farbraumbereich 20 mit viel Hintergrundlicht und wenig Fluoreszenzlicht und durch den Bereich 21 mit viel Fluoreszenzlicht und wenig Hintergrundlicht. Auf dieser Geraden F1 lassen sich also zwischen den Farbraumbereichen 20 und 21 unterschiedliche Fluoreszenzwerte darstellen. Die Projektion eines Farbvektors auf die Gerade F1 liefert somit ein Maß für die Fluoreszenz.

Fig. 2 zeigt die Bildverarbeitung 5 im Detail. Sie weist drei Stufen 8, 9, 10 auf, in denen die Pixel des Bildes nacheinander verarbeitet werden.

In der ersten Stufe 8 wird pixelweise aus dem von der Kamera verwendeten Bildraum, der in der Regel ein RGB-Farbraum ist, in einen anderen Farbraum umgerechnet, der mit FXY bezeichnet wird. Die Koordinatenachsen X und Y sind unerheblich. Sie müssen nur so gewählt werden, dass mit F, X und Y ein dreidimensionaler Farbraum aufgespannt wird. Wichtig ist die Lage der Koordinatenachse F, die parallel zur Geraden F1 der Fig. 5 gelegt werden muss und die somit die Fluoreszenzkomponente eines Pixels im neuen Farbraum FXY angibt. Der Farbraum FXY geht aus dem ursprünglichen Farbraum RGB also durch Drehung und gegebenenfalls Verschiebung hervor.

Die zweite Bildverarbeitungsstufe 9 dient zur nichtlinearen Veränderung der Fluoreszenzwerte. Anschließend wird in der Stufe 10 vom FXY-Farbraum auf einen üblicherweise zur Bilddarstellung verwendeten Farbraum umgerechnet, was zumeist wiederum der RGB-Farbraum ist.

Fig. 3 zeigt in genauerer Darstellung die Kennlinie 13, die in der Bildverarbeitungsstufe 9 zur Beeinflussung der Fluoreszenzwerte verwendet wird. Das Ziel dieser Umrechnung ist eine Verbesserung der Sichtbarkeit des Fluoreszenzlichtes, das sehr schwach ist. Dabei sollen die übrigen Bildeigenschaften möglichst wenig verändert werden. Dies wird dadurch erreicht, dass die Bildumrechnung nur auf der F-Koordinate erfolgt, die von den übrigen Koordinaten unabhängig ist. Es kann also die Fluoreszenz sehr stark beeinflusst werden, ohne den Bildeindruck ansonsten zu ändern.

Bei der Beeinflussung der Fluoreszenz wird die in Fig. 3 beispielhaft dargestellte Kennlinie 13 verwendet. An den beiden Enden der Kennlinie, also in den Bereichen 0 bis a, wo deutlich erkennbares blaues Licht vorliegt, oder im Bereich c bis 1, wo deutlich erkennbares Fluoreszenzlicht (rot) vorliegt, wird nichts verändert. In dem dazwischen liegenden Bereich a bis c wird im unteren Abschnitt a bis b, also im schwach blauen Bereich, die Fluoreszenz abgesenkt, also der Blauanteil verstärkt, während im oberen Abschnitt von b bis c die Fluoreszenz (rot) angehoben wird.

Das bedeutet, dass in Bereichen mit geringen, schwer erkennbaren Fluoreszenzanteilen, der Fluoreszenzkontrast erhöht wird. Mit dieser Anordnung wird vor allem in den Bereichen mit mittlerer Fluoreszenz, in denen schwer zu erkennen ist, ob hier nun Fluoreszenz vorliegt, oder nicht, die Aussage des Bildes verbessert.

Im besprochenen Ausführungsbeispiel liegt das Hintergrundlicht 12 im Blauen und das Fluoreszenzlicht 11 im Roten. Die Farbe der Fluoreszenz kann je nach verwendetem Fluoreszenzfarbstoff auch anders liegen. Auch das Hintergrundlicht kann anders gewählt werden, sofern es nur einem einigermaßen umgrenzten Raumbereich im Farbraum entspricht.

Die zur Markierung des darzustellenden Tumors verwendete fluoreszierende Substanz kann mit der Zeit in der Wirkung nachlassen, so daß sich die Fluoreszenz verringert. Um dies auszugleichen kann die Kennlinie 13 derart mit der Zeit verändert werden, daß entsprechend dem zeitlichen Ausbleichen bzw. Nachlassen der Substanz der Fluoreszenzkontrast erhöht wird, so daß im Ergebnis der Fluoreszenzeindruck im Wesentlichen gleich bleibt.

## Patentansprüche

1. Verfahren zur Verbesserung von mit einer Farb-Videokamera (3) aufgenommenen Endoskopbildem von mit Hintergrundlicht (12) beleuchteten, stellenweise fluoreszierenden Gewebebereichen, **dadurch gekennzeichnet, dass** die Farbpixel der Endoskopbilder von dem Farbraum (RGB) der Videokamera (3) in einen Farbraum (FXY) transformiert werden, in dem eine Gerade (F1), die durch den Farbraumbereich (21) des Fluoreszenzlichtes (11) und durch den Farbraumbereich (20) des Hintergrundlichtes (12) verläuft, parallel zu einer die Fluoreszenzkomponente der Pixel beschreibenden Koordinatenachse (F) verläuft, und dass in diesem Farbraum (FXY) die Fluoreszenzkomponente der Pixel mit einer nichtlinearen Kennlinie (13) verändert wird, die den Fluoreszenzkontrast, nämlich die Differenz zwischen höheren Fluoreszenzwerten und niedrigeren Fluoreszenzwerten wenigstens bereichsweise vergrößert, und dass schließlich die Pixel in einen zur Bilddarstellung geeigneten Farbraum (RGB) transformiert werden.

2. Verfahren nach Anspruch 1, **dadurch gekennzeichnet, dass** die Kennlinie (13) derart ausgebildet ist, dass sich in den oberen und unteren Endbereichen (0 - a, c - 1) keine Veränderung ergibt und sich in dem dazwischen liegenden Bereich (a - c) in einem oberen Abschnitt (b - c) der Kennlinie (13) eine Verstärkung und in einem unteren Abschnitt (a - b) eine Abschwächung ergibt.

3. Verfahren nach Anspruch 1, **dadurch gekennzeichnet, dass** die Kennlinie (13) derart zeitlich verändert wird, dass sich der Fluoreszenzkontrast mit der Zeit erhöht.

4. Medizinisches Endoskop (1) mit einer Farb-Videokamera (3), einer daran angeschlossenen Bildverarbeitungseinrichtung (5) und einer daran angeschlossenen Bilddarstellungseinrichtung (7), **dadurch gekennzeichnet, dass** die Bildverarbeitungseinrichtung (5) zur Durchführung des Verfahrens nach einem der vorhergehenden Ansprüche ausgebildet ist.

## Claims

1. Method for improving endoscope images of background light (12)-illuminated, partly fluorescent tissue regions, which have been recorded with a colour video-camera (3), **characterised in that** the colour pixels of the endoscope images are transformed from the colour space (RGB) of the video-camera (3) to a colour space (FXY), in which a straight line (F1) extending through the colour space region (21) of the fluorescence light (11) and through the colour space region (20) of the background light (12) extends parallel to a coordinate axis (F) that describes the fluorescence component of the pixels, and **in that** the fluorescence component of the pixels is changed in said colour space (FXY) through a non-linear characteristic line that increases, at least partly, the fluorescence contrast, namely the difference between higher fluorescence values and lower fluorescence values, and **in that** the pixels are finally transformed into a colour space (RGB) that is suitable for image display.

2. Method according to claim 1, **characterised in that** the characteristic line (13) is designed such that no change results in the upper and lower end-regions (0 - a, c - 1) and **in that**, in the region situated in between these, an amplification results (a - c) in an upper section (b - c) of the characteristic curve (13) and an attenuation results in a lower section (a - b).

3. Method according to claim 1, **characterised in that** the characteristic line (13) is changed over time such that the fluorescence contrast increases over time.

4. Medical endoscope (1) comprising a colour video-camera (3), an image processing facility (5) connected thereto, and an image displaying facility (7) connected thereto, **characterised in that** the image processing facility (5) is designed for implementing the method according to any one of the preceding claims.

## Revendications

1. Procédé destiné à améliorer les images endoscopiques enregistrées par une caméra vidéo couleur (3) de régions de tissus partiellement fluorescentes éclairées par rétroéclairage (12), **caractérisé en ce que** les pixels couleur des images endoscopiques de l'espace colorimétrique (RGB) de la caméra vidéo (3) sont transformés vers un espace colorimétrique (FXY) dans lequel une droite (FI) qui traverse la région (21) de l'espace colorimétrique de la lumière fluorescente (11) et la région (20) de l'espace colorimétrique de la lumière de rétroéclairage (12), est parallèle à un axe de coordonnées (F) décrivant la composante fluorescente des pixels, et **en ce que** dans cet espace colorimétrique (FXY) la composante fluorescente des pixels est modifiée avec une caractéristique (13) non linéaire qui augmente dans certaines régions au moins le contraste de fluorescence, à savoir la différence entre les intensités de fluorescence élevées et les faibles intensités de fluorescence, et enfin **en ce que** les pixels sont transformés vers un espace colorimétrique (RGB) approprié à la représentation de l'image.

2. Procédé selon la revendication 1, **caractérisé en ce que** la forme de la caractéristique (13) est telle qu'il n'y a pas de modification dans les zones d'extrémité supérieure et inférieure (0 - a, c - 1) et que, dans la zone intermédiaire (a - c), il en résulte dans une section supérieure (b - c) de la caractéristique (13) une amplification et dans une section inférieure (a - b) une atténuation.

3. Procédé selon la revendication 1, **caractérisé en ce que** la caractéristique (13) est modifiée dans le temps de façon telle que le contraste de fluorescence augmente dans le temps.

4. Endoscope médical (1) avec une caméra vidéo couleur (3) et, connectés à celle-ci, un dispositif de traitement de l'image (5) et un dispositif de représentation de l'image (7), **caractérisé en ce que** le dispositif de dispositif de traitement de l'image (5) est conçu de façon à permettre l'application du procédé selon l'une des revendications précédentes.
